Europäisches Patentamt

European Patent Office

Office européen des brevets

(19)

(11) **EP 1 566 760 A2**

(12) ## EUROPEAN PATENT APPLICATION

(43) Date of publication:
**24.08.2005 Bulletin 2005/34**

(51) Int Cl.⁷: **G06F 19/00**

(21) Application number: **05003696.1**

(22) Date of filing: **21.02.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **21.02.2004 KR 2004011653**

(71) Applicant: **SAMSUNG ELECTRONICS CO., LTD. Gyeonggi-do (KR)**

(72) Inventors:
• **Oh, Ji-young**
  **Yeongtong-gu Suwon-si Gyeonggi-do (KR)**
• **Kim, Kyoung-a**
  **Gwangmyeong-si Gyeonggi-do (KR)**
• **Nam, Yun-sun**
  **Seongnam-si Gyeonggi-do (KR)**
• **Lee, Jeong-gun**
  **Seocho-gu Seoul (KR)**

(74) Representative: **Grünecker, Kinkeldey, Stockmair & Schwanhäusser Anwaltssozietät Maximilianstrasse 58 80538 München (DE)**

(54) **Method and system of replacing missing genotyping data**

(57)    A system and method of replacing a missing genotyping data are provided. The method includes: constructing a sample group consisting of a genotyping data with respect to SNP sites of at least one gene sample; comparing a similarity between a sample of the sample group having a missing genotyping data of an SNP site with the other samples of the sample group, and selecting a predetermined number of the samples in order of high similarity; and checking a genotyping data having a greatest frequency occurring in an SNP site disposed at the same position as the SNP site having the missing genotyping data among the selected samples, and replacing the missing genotyping data with the genotyping data having the greatest frequency. It is possible to prevent an incorrect analysis result due to data mismatch, which is caused by data loss.

FIG. 1

```
                    START
                      │
  CONSTRUCT SAMPLE GROUP CONSISTING OF
GENOTYPING DATA WITH RESPECT TO SNP SITES OF SAMPLES   — S100
                      │
     SELECT PREDETERMINED NUMBER OF SAMPLES
   BASED ON SIMILARITY BETWEEN SAMPLE HAVING           — S110
  MISSING GENOTYPING DATA AND REMAINING SAMPLES
                      │
  REPLACING MISSING GENOTYPING DATA WITH GENOTYPING
       DATA HAVING GREATEST FRQUENCY                    — S120
                      │
                    END
```

EP 1 566 760 A2

**Description**

BACKGROUND OF THE INVENTION

1. Field of the Invention

The present invention relates to a method and system of replacing a missing genotyping data, and more particularly, to a method and system of replacing a missing genotyping data of a specific SNP site among samples having SNP sites.

2. Description of the Related Art

Developments using genetic information have been made in various manners, focusing on health and welfare of mankind. Men have different genes and thus it can be said that men have different genetic characters. The difference of genetic character is determined by a single nucleotide polymorphism (SNP). The SNP forms a new gene group due to a single nucleotide change in the gene and represents a change in a genetic character of the group. Accordingly, the SNP exhibits local and racial differences.

However, samples of patients or normal persons having SNP sites can include SNP sites in which genotyping data are missing due to an error of an operator or an inaccuracy of test data during the test.

In this case, an analysis process is performed after removing the samples with SNP sites in which genotyping data are missing, or after removing SNP sites in which genotyping data are missing. Accordingly, a large amount of data may be lost and an incorrect result may be caused due to a shortage of the number of patient samples and normal samples.

[Table 1]

|  | A1A1 | A1A2 | A2A2 |
|---|---|---|---|
| Normal | 3 | 200 | 30 |
| Patient | 1 | 100 | 90 |

As shown in Table 1, types A1A1 of the normal person and the patient in a specific SNP site is 3:1. That is, there is a significant difference in types A1A1 of them. However, if the sample is removed in a state that genotyping data of some SNP sites among the normal samples are missing, the number of normal samples may be "1". At this time, types of both the normal person and the patient of the corresponding SNP site become equal to 1:1, such that even a chi-test may analyze that there is no significant difference.

Most of discrimination algorithms or clustering algorithm, such as SVD, Logistic regression, PCA and SOM, receive a full matrix type data as an input variable. The full matrix type includes no element in which data is empty. Accordingly, a large amount of data is lost by removing a column or row corresponding to the element in which data is empty, resulting in an incorrect result.

If the column or row of an element having a missing genotyping data is removed, values representative of dataset characteristics, such as MSE or mean value, are changed. Therefore, there occurs a problem in that a type analysis result of an entire data is changed.

SUMMARY OF THE INVENTION

The present invention provides a system and method of replacing a missing genotyping data, in which an incorrect analysis result due to a large quantity of data loss can be prevented by replacing data of SNP site having a missing genotyping data with data occurring frequently in a similar group.

Also, the present invention provide a computer-readable recording medium, in which an incorrect analysis result due to a large quantity of data loss can be prevented by replacing data of SNP site having a missing genotyping data with data occurring frequently in a similar group.

According to an aspect of the present invention, a method of replacing a missing genotyping data includes: constructing a sample group consisting of a genotyping data with respect to SNP sites of at least one gene samples; comparing a similarity between a sample of the sample group having a missing genotyping data of an SNP site with the other samples of the sample group, and selecting a predetermined number of the samples in order of high similarity; and checking a genotyping data having a greatest frequency occurring in an SNP site disposed at the same position as the SNP site having the missing genotyping data among the selected samples, and replacing the missing genotyping data with the genotyping data having the greatest frequency.

According to another aspect of the present invention, a system of replacing a missing genotyping data includes: a sample group constructing unit constructing sample groups consisting of genotyping data with respect to SNP sites of at least one gene sample; a similarity comparing unit comparing a similarity between a sample of the sample group having a missing genotyping data of an SNP site with the other samples of the sample group and selecting a predetermined number of samples in order of high similarity; and a data replacing unit checking a genotyping data having a greatest frequency occurring in an SNP site disposed at the same position the SNP site having the missing genotyping data among the selected samples, and replacing the missing genotyping data with the genotyping data having the greatest frequency.

Accordingly, it is possible to prevent an incorrect analysis result due to data mismatch, which is caused by data loss.

BRIEF DESCRIPTION OF THE DRAWINGS

The above and other features and advantages of the present invention will become more apparent by describing in detail exemplary embodiments thereof with reference to the attached drawings in which:

FIG. 1 is a flowchart illustrating a method of replacing a missing genotyping data according to an embodiment of the present invention;

FIG. 2 is a flowchart illustrating a method of replacing a missing genotyping data according to an embodiment of the present invention;

FIG. 3 is a view of a sample group consisting of genotyping data;

FIG. 4 is a view illustrating a coding of genotyping data into numerical data;

FIG. 5 is a view of a comparison result of similarity between samples;

FIG. 6 is a view illustrating a method of finding data to be substituted for a missing data based on the comparison result of the similarity; and

FIG. 7 is a view illustrating a system of replacing a missing genotyping data according to the present invention.

DETAILED DESCRIPTION OF THE INVENTION

The present invention will now be described more fully with reference to the accompanying drawings.

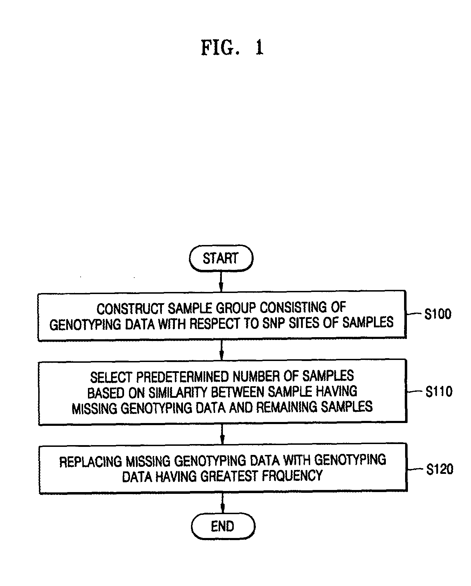

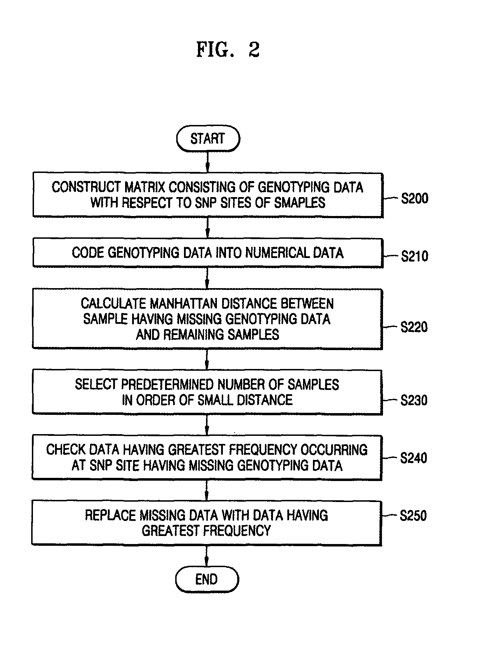

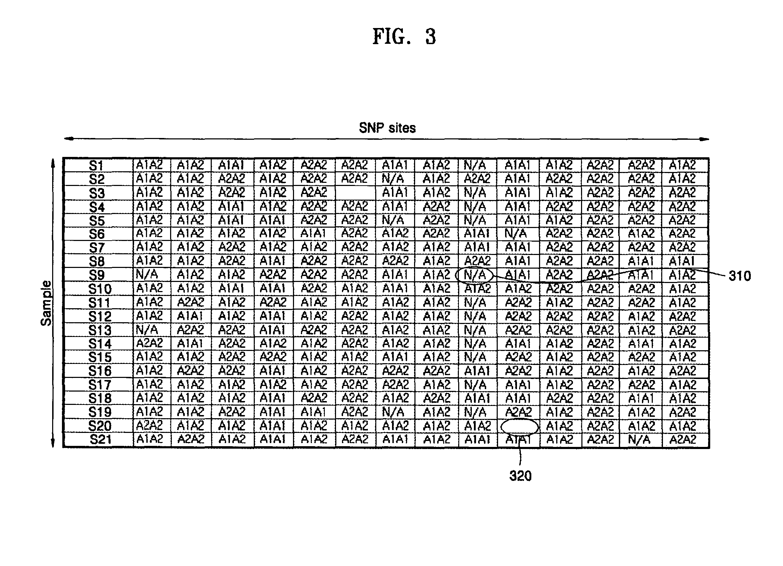

First, flowcharts of FIGS. 3 to 6 will be described and then a method of replacing a missing genotyping data according to the present invention will be described with reference to FIGS. 1 and 2.

FIG. 3 is a view of a sample group consisting of genotyping data.

Referring to FIG. 3, each row represents one patient or one normal person, and each column represents respective SNP sites. When there is no test data 320 regarding a specific SNP site or when a test data cannot be used due to an incorrect result 310, a genotyping data does not exist in the corresponding SNP site.

According to the prior art, a large amount of data is lost by removing samples (rows of S9 and S20 in FIG. 3) including SNP sites in which the genotyping data do not exist, or by removing columns of SNP sites 310 and 320 in which the genotyping data do not exist. In most cases, an incorrect result is caused by a shortage of the number of the patient samples and the normal samples.

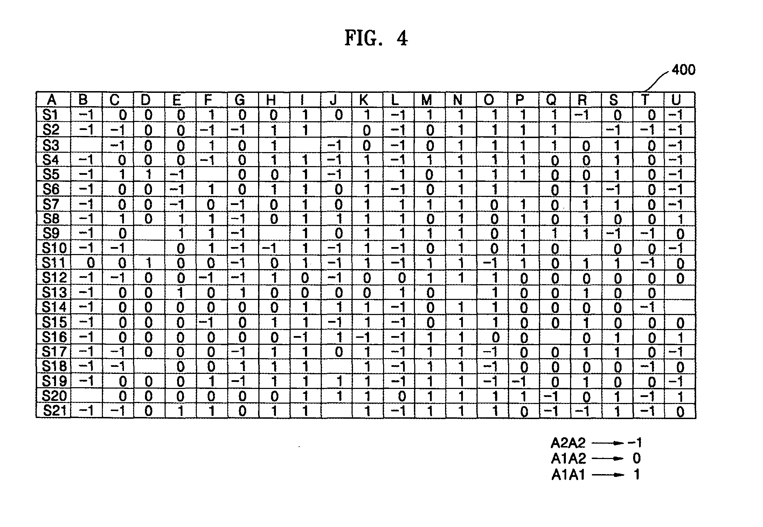

FIG. 4 illustrates genotyping data coded into numeric data.

Referring to FIG. 4, in a sample group 400 constructed with genotyping data regarding SNP sites, a genotyping data of A2A2 is replaced with "-1" and a genotyping of A1A2 is replaced with "0". Also, a genotyping data of A1A1 is replaced with "1". SNP sites having no genotyping data are represented by blank.

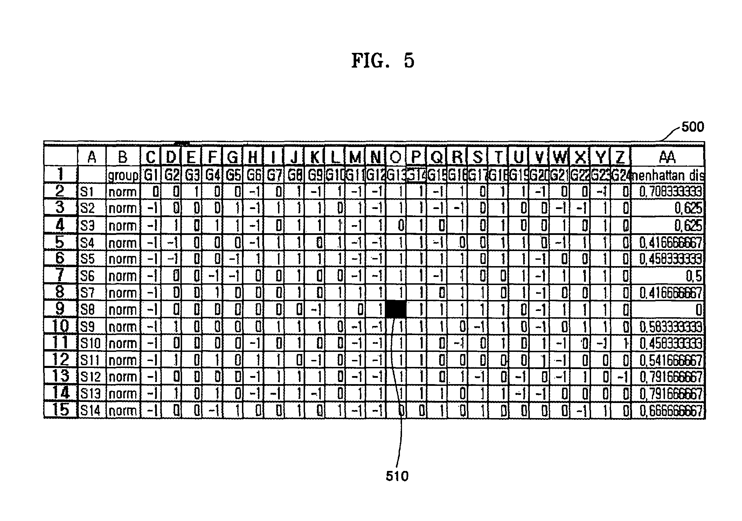

FIG. 5 illustrates comparison results of similarity with respect to the samples.

Referring to FIG. 5, a sample (a ninth column) in which a genotyping data of a specific SNP site is missing is compared with the remaining samples. FIG. 5 shows a result (a column AA) obtained when a manhattan distance method is used as a method of comparing the similarity.

For example, there is a sample of a row S8 in which a genotyping data of an SNP site 510 of a column O is missing. Manhattan distances between the sample of the row S8 and the remaining samples are calculated. The predetermined number of the samples is selected based on the calculated manhattan distance. Using the selected samples, a value to be substituted for a value of the missing genotyping data 510 is calculated.

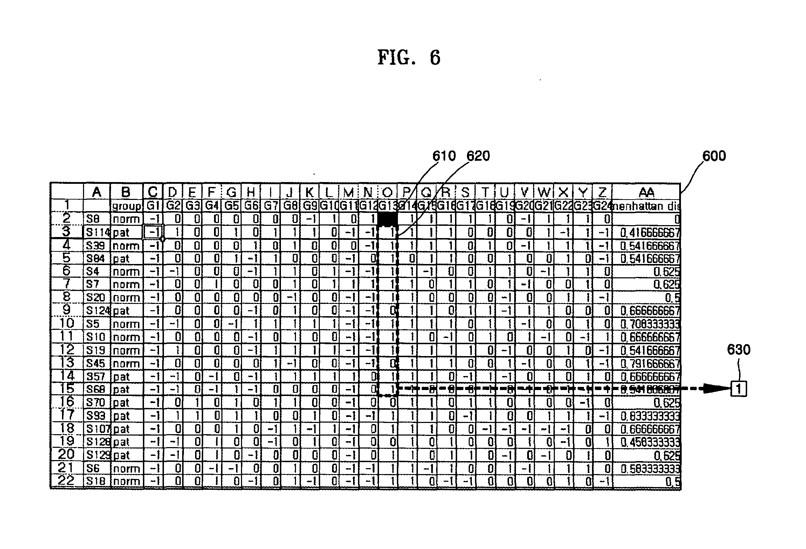

FIG. 6 illustrates a process of finding data to be substituted for the missing data based on the comparison result of the similarity.

Referring to FIG. 6, a similarity (manhattan distance) between the sample (the row S8) having a missing genotyping data of a specific SNP site and the remaining samples is compared and a predetermined number of samples are selected in order of high similarity (in order of small manhattan distance). Data values of the SNP sites of the selected samples contained in the same column (column O) as the missing SNP site are examined to find data value having the greatest frequency. Then, the missing genotyping data is replaced with the data value of the greatest frequency.

For example, thirteen samples are selected in order of small manhattan distance, and data values 620 of the SNP sites in the column O of the selected samples are compared. In the data of the column O of the selected samples, there are eleven 1s and two 0s. Accordingly, the greatest frequency 630 is 1 and the missing data 610 is replaced with 1.

FIG. 1 is a flowchart illustrating a method of replacing the missing genotyping data according to an embodiment of the present invention.

Referring to FIG. 1, a sample group (refer to FIG. 3) consisting of the genotyping data regarding the SNP sites of at least one sample is constructed (S100). The sample group is configured in matrix. Rows represent respective samples and columns represent respective SNP sites. Each component of the matrix consisting of the SNP sites is one of three genotyping data A1A2, A1A1 and A2A2. If there is no test data for a specific SNP site in each sample, a component of the corresponding SNP site is represented by a blank. Also, if there is a test data but it cannot be used due to an incorrect result, a component of the corresponding SNP site is represented by an N/A 310 or blank 320.

As the number of empty genotyping data is increasing, a large amount of data may be lost during an analysis process of judging SNP sites that can distinguish a patient group from a normal group. In most cases, a shortage of the number of the patient groups and the normal samples occurs and thus an incorrect result is caused.

In the sample group expressed in matrix, a similarity between the sample containing the component with the missing genotyping data and the remaining samples is compared (S110). Then, a predetermined number of samples are selected in order of high similarity (S110).

Manhattan distance method is used as a method of comparing the similarity between the samples. The manhattan distance method is used to calculate distance of categorical data. In an equation of obtaining manhattan distance, the respective samples are treated in vector type and it is named a sample vector. Following equations 1 through 3 represent a sample vector and manhattan distance.

[Equation 1]

$$S1(x11, x12, x13,...,x1n), S2(x21, x22, x213,...,x2n),..., Sn(xn1, xn2, xn3,..., xnn)$$

where, S1, S2 and Sn represent sample vectors for the respective samples, and the respective components x11, ... , xnn of the sample vectors correspond to data values of SNP sites of the respective samples.

[Equation 2]

$$\text{Distance between sample 1 and sample 2} = (|x11-x21|n+|x12-x22|n+$$

$$...+|x13-x23|n +...+ |x1n- x2n|n)1/n$$

where, x11, x21,..., x2n represent the respective components of the sample vectors S1 and S2.

[Equation 3]

$$\text{Distance between sample 1 and sample 2} = (|x11- x21| + |x12 - x22| +$$

$$|x13 - x23| +... +|x1n - x2n|)/n$$

where, x11, x21, ... , x2n represent the respective components of the sample vectors S1 and S2.

In addition to the manhattan distance, other methods for calculating the similarity between the samples are proposed. Equation 4 represents an eucldeian method, equation 5 a correlation method, equation 6 a canberra metric (dissimilarity coefficienct), equation 7 a jaccard's coeffient II (similarity coefficient), equation 8 a city block distance, equation 9 a squared euclidean measure, equation 10 a cheby chev distance, respectively. In equations 4 through 10, "g" and "g*" represent groups to be compared, and "gi" and "g*|" represent components of the groups.

[Equation 4]

$$d(q,r)_{g,g'} = \left[\sum_i \left|x_{gi} - x_{g*i}\right|^r\right]^{1/q}$$

[Equation 5]

$$r_{g,g*} = \frac{\sum_i (x_{gi} - \bar{x}_g)\cdot(x_{g*i} - \bar{x}_{g*i})}{[\sum_i (x_{gi} - \bar{x}_g)^2 \cdot \sum_i (x_{g*i} - \bar{x}_{g*})^2]^{1/2}}$$

[Equation 6]

$$Canberra_{g,g*} = \sum_i \frac{\left|x_{g,i} - x_{g*,i}\right|}{(x_{g,i} + x_{g*,i})}$$

[Equation 7]

$$Jaccard-II_{g,g*} = \frac{\sum_i x_{gi} + \sum_i x_{g*i} - 2\sum_i \min(x_{gi,}x_{g*i})}{\sum_i x_{gi} + \sum_i x_{g*i} - \sum_i \min(x_{gi},x_{g*i})}$$

[Equation 8]

$$CITY_{g,g*} = d(r=1,q=1)_{g,g*} = \sum_i \left|x_{gi} - x_{g*i}\right|$$

[Equation 9]

$$QEUKLID_{g,g*} = d(r=2,q=1) = \sum_i (x_{g,i} - x_{g*i})^2$$

[Equation 10]

$$CHEBYCHEV_{g,g*} = d(r=\infty, q=\infty)_{g,g*} = \max|x_{gi} - x_{g*i}|$$

Equations 1 through 10 are examples of methods for calculating the similarity between two groups and another methods can also be applied.

Samples are selected based on the similarity between a sample having a missing genotyping data and the remaining samples (S110). Among the selected samples, a frequency of genotyping data existing in SNP sites located at the same position as the SNP site having the missing genotyping data is examined (S120). A genotyping data having the greatest frequency is allocated as a genotyping data of a missing SNP site (S120).

In other words, using data of a group having a similar profile, an empty data is replaced with a genotyping data frequently occurring in the similar group. When a plurality of SNP sites are identical between the samples, it is considered as the samples having the similar profile and it is considered as one group. It is similar to an assumption of a clustering of a gene expression data, which analyzes a gene expression, or a discrimination analysis of a genotyping data.

FIG. 2 is a flowchart illustrating a method of replacing a missing genotyping data according to another embodiment of the present invention.

Referring to FIG. 2, respective rows are constituted with samples of patient or normal person, and respective columns are constituted with SNP sites (S200). A component of the matrix, which is a specific SNP site of a specific sample, includes genotyping data. The genotyping data is combined with two homos and one hetero according to a combination of gene. A matrix consisting of genotyping data is shown in FIG. 3.

The genotyping data constituting the respective components of the matrix are coded into numerical data (S210, in FIG. 4). That is, the numerical data correspond one-to-one with the respective genotyping data.

Among the samples constructed with SNP sites, there exist samples whose genotyping data of a specific SNP site is missing. In this case, a conventional analysis is performed by removing the samples having the missing genotyping data or entirely removing the specific SNP site having the missing genotyping data. As a result, an incorrect analysis result is caused.

According to the present invention, the missing genotyping data are replaced with a specific genotyping data so as to prevent the incorrect analysis result.

First, manhattan distance between the sample having the missing genotyping data of the specific SNP site and the remaining samples is calculated (S220, in FIG. 5). Samples with smaller manhattan distance have high similarity to the sample having the missing genotyping data. The remaining samples whose similarity is compared with the sample having the missing genotyping data are perfect samples having no missing genotyping data. If there is the sample having the missing genotyping data of the specific SNP site among the remaining samples, the similarity in only the remaining perfect samples having no missing genotyping data is compared.

After calculating the manhattan distance, a predetermined number of samples are selected in order of small distance (S230, in FIG. 6). As the number of the selected samples is larger, a more accurate replacement value can be selected. However, if the number of the selected samples is more than a predetermined value, there is almost no difference in the accuracy. Accordingly, the number of samples to be selected is determined based on a test data.

If the samples are selected, the samples are examined to check what is the genotyping data of the SNP site in the same as the column of the specific SNP site having the missing genotyping data (S240, in FIG. 6).

If the genotyping data having the greatest frequency is checked through the examination (S240), the SNP site of the sample having the missing genotyping data is replaced with the genotyping data having the greatest frequency (S250).

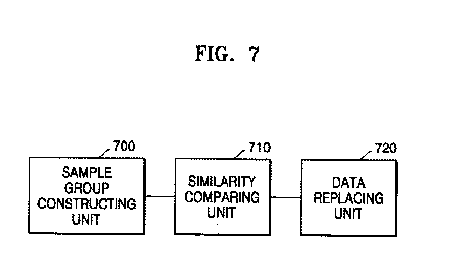

FIG. 7 is a block diagram illustrating the system of replacing the missing genotyping data according to the present invention.

Referring to FIG. 7, the system includes a sample group constructing unit 700, a similarity comparing unit 710, and a data replacing unit 720.

The sample group constructing unit 700 constructs sample groups consisting of genotyping data with respect to the SNP sites of at least one genotyping sample. In the sample group, rows represent samples and columns represent SNP sites.

The similarity comparing unit 710 compares the samples having missing genotyping data of the SNP site with the remaining samples. The similarity comparing unit 710 uses manhattan distance for similarity comparison. Also, as described in FIG. 1, a variety of methods can be applied for the similarity comparison.

The data replacing unit 720 checks genotyping data of the greatest frequency occurring in the SNP sites disposed at the same position as the SNP site having the missing genotyping data among the samples selected by the similarity comparing unit 710. Also, the data replacing unit 720 replaces the missing genotyping data with the genotyping data having the greatest frequency occurring in the selected samples.

The invention can also be embodied as computer readable codes on a computer-readable recording medium. The computer-readable recording medium is any data storage device that can store data which can be thereafter read by a computer system. Examples of the computer-readable recording medium include read-only memory (ROM), random-access memory (RAM), CD-ROMs, magnetic tapes, floppy disks, optical data storage devices, and carrier waves (such as data transmission through the Internet). The computer-readable recording medium can also be distributed over network coupled computer systems so that the computer readable code is stored and executed in a distributed fashion.

While the present invention has been particularly shown and described with reference to exemplary embodiments thereof, it will be understood by those of ordinary skill in the art that various changes in form and details may be made therein without departing from the spirit and scope of the present invention as defined by the following claims.

**Claims**

**1.** A method of replacing a missing genotyping data, comprising:

constructing a sample group consisting of a genotyping data with respect to SNP sites of at least one gene samples;

comparing a similarity between a sample of the sample group having a missing genotyping data of an SNP site with the other samples of the sample group, and selecting a predetermined number of the samples in order of high similarity; and

checking a genotyping data having a greatest frequency occurring in an SNP site disposed at the same position as the SNP site having the missing genotyping data among the selected samples, and replacing the missing genotyping data with the genotyping data having the greatest frequency.

**2.** The method of claim 1, wherein the similarity is compared using one of a manhattan distance method, an eucledian method, a correlation method, a canberra metric method, a jaccard's coefficient II method, a city block distance method, a squared eucidean measure method, and a cheby chev distance method.

**3.** The method of claim 1, wherein the operation of selecting the samples comprises selecting samples whose SNP sites are not missing.

**4.** The method of claim 1, wherein the construction of the sample group comprises constructing genotyping data having three types according to combination characteristics of gene in a matrix form represented with numerical values corresponding to the respective genotyping data.

**5.** The method of claim 4, wherein the construction of the sample group comprises representing the genotyping data using numerical data of -1, 0 and 1.

**6.** The method of claim 1, wherein the construction of the sample group comprises representing an SNP site having an absence of test data or an incorrect result of test data using blanks.

**7.** A system of replacing a missing genotyping data, comprising:

a sample group constructing unit constructing sample groups consisting of genotyping data with respect to SNP sites of at least one gene sample;

a similarity comparing unit comparing a similarity between a sample of the sample group having a missing genotyping data of an SNP site with the other samples of the sample group and selecting a predetermined number of samples in order of high similarity; and

a data replacing unit checking a genotyping data having a greatest frequency occurring in an SNP site disposed at the same position the SNP site having the missing genotyping data among the selected samples, and replacing the missing genotyping data with the genotyping data having the greatest frequency.

**8.** The system of claim 7, wherein the similarity comparing unit compares the similarity between the samples using a manhattan distance method.

**9.** A computer-readable recording medium encoded with processing instructions for implementing a method of replacing a missing genotyping data, the method comprising:

constructing a sample group consisting of a genotyping data with respect to SNP sites of at least one gene samples;

comparing a similarity between a sample of the sample group having a missing genotyping data of an SNP site with the other samples of the sample group, and selecting a predetermined number of the samples in order of high similarity; and

checking a genotyping data having a greatest frequency occurring in an SNP site disposed at the same position as the SNP site having the missing genotyping data among the selected samples, and replacing the missing genotyping data with the genotyping data having the greatest frequency.

## FIG. 1

```
                    ┌─────────┐
                    │  START  │
                    └────┬────┘
                         │
                         ▼
┌──────────────────────────────────────────────────────┐
│        CONSTRUCT SAMPLE GROUP CONSISTING OF           │─── S100
│ GENOTYPING DATA WITH RESPECT TO SNP SITES OF SAMPLES  │
└──────────────────────────┬───────────────────────────┘
                           │
                           ▼
┌──────────────────────────────────────────────────────┐
│         SELECT PREDETERMINED NUMBER OF SAMPLES        │─── S110
│       BASED ON SIMILARITY BETWEEN SAMPLE HAVING       │
│      MISSING GENOTYPING DATA AND REMAINING SAMPLES    │
└──────────────────────────┬───────────────────────────┘
                           │
                           ▼
┌──────────────────────────────────────────────────────┐
│   REPLACING MISSING GENOTYPING DATA WITH GENOTYPING   │─── S120
│          DATA HAVING GREATEST FRQUENCY               │
└──────────────────────────┬───────────────────────────┘
                           │
                           ▼
                    ┌─────────┐
                    │   END   │
                    └─────────┘
```

# FIG. 2

```
                        ┌─────────┐
                        │  START  │
                        └─────────┘
                             │
                             ▼
        ┌──────────────────────────────────────────┐
        │ CONSTRUCT MATRIX CONSISTING OF GENOTYPING │── S200
        │ DATA WITH RESPECT TO SNP SITES OF SMAPLES │
        └──────────────────────────────────────────┘
                             │
                             ▼
        ┌──────────────────────────────────────────┐
        │ CODE GENOTYPING DATA INTO NUMERICAL DATA  │── S210
        └──────────────────────────────────────────┘
                             │
                             ▼
        ┌──────────────────────────────────────────┐
        │     CALCULATE MANHATTAN DISTANCE BETWEEN  │
        │      SAMPLE HAVING MISSING GENOTYPING DATA│── S220
        │           AND REMAINING SAMPLES           │
        └──────────────────────────────────────────┘
                             │
                             ▼
        ┌──────────────────────────────────────────┐
        │    SELECT PREDETERMINED NUMBER OF SAMPLES │── S230
        │         IN ORDER OF SMALL DISTANCE        │
        └──────────────────────────────────────────┘
                             │
                             ▼
        ┌──────────────────────────────────────────┐
        │ CHECK DATA HAVING GREATEST FREQUENCY      │── S240
        │ OCCURRING AT SNP SITE HAVING MISSING      │
        │ GENOTYPING DATA                           │
        └──────────────────────────────────────────┘
                             │
                             ▼
        ┌──────────────────────────────────────────┐
        │    REPLACE MISSING DATA WITH DATA HAVING  │── S250
        │            GREATEST FREQUENCY             │
        └──────────────────────────────────────────┘
                             │
                             ▼
                        ┌─────────┐
                        │   END   │
                        └─────────┘
```

FIG. 3

SNP sites

Sample

| | | | | | | | | | | | | | | |
|---|---|---|---|---|---|---|---|---|---|---|---|---|---|---|
| S1 | A1A2 | A1A2 | A1A1 | A1A2 | A2A2 | A2A2 | A1A1 | A1A2 | N/A | A1A1 | A1A2 | A2A2 | A2A2 | A1A2 |
| S2 | A1A2 | A1A2 | A2A2 | A1A2 | A2A2 | A2A2 | N/A | A1A2 | A2A2 | A1A1 | A2A2 | A2A2 | A2A2 | A1A2 |
| S3 | A1A2 | A1A2 | A2A2 | A1A2 | A2A2 | | A1A1 | A1A2 | N/A | A1A1 | A1A2 | A2A2 | A2A2 | A2A2 |
| S4 | A1A2 | A1A2 | A1A1 | A1A2 | A2A2 | A2A2 | A1A1 | A2A2 | N/A | A1A1 | A2A2 | A2A2 | A2A2 | A2A2 |
| S5 | A1A2 | A1A2 | A1A1 | A1A1 | A2A2 | A2A2 | N/A | A2A2 | N/A | A1A1 | A1A2 | A2A2 | A2A2 | A2A2 |
| S6 | A1A2 | A1A2 | A1A2 | A1A2 | A1A1 | A2A2 | A1A2 | A2A2 | A1A1 | N/A | A2A2 | A2A2 | A1A2 | A2A2 |
| S7 | A1A2 | A1A2 | A2A2 | A1A2 | A1A2 | A2A2 | A1A2 | A1A2 | A1A1 | A1A1 | A2A2 | A2A2 | A2A2 | A2A2 |
| S8 | A1A2 | A1A2 | A2A2 | A1A1 | A2A2 | A2A2 | A2A2 | A1A2 | A2A2 | A1A1 | A2A2 | A2A2 | A1A1 | A1A1 |
| S9 | N/A | A1A2 | A1A2 | A2A2 | A2A2 | A2A2 | A1A1 | A1A2 | N/A | A1A1 | A2A2 | A2A2 | A1A1 | A1A2 |
| S10 | A1A2 | A1A2 | A1A1 | A1A2 | A2A2 | A1A2 | A1A1 | A1A2 | A1A2 | A1A2 | A2A2 | A2A2 | A2A2 | A1A2 |
| S11 | A1A2 | A2A2 | A1A2 | A2A2 | A1A2 | A1A1 | A1A2 | A1A2 | N/A | A2A2 | A1A2 | A2A2 | A2A2 | A1A2 |
| S12 | A1A2 | A1A1 | A1A2 | A1A1 | A1A2 | A2A2 | A1A2 | A1A2 | N/A | A2A2 | A2A2 | A2A2 | A1A2 | A2A2 |
| S13 | N/A | A2A2 | A2A2 | A1A1 | A2A2 | A2A2 | A1A2 | A1A2 | N/A | A2A2 | A2A2 | A2A2 | A1A2 | A2A2 |
| S14 | A2A2 | A1A1 | A2A2 | A1A2 | A1A2 | A2A2 | A1A2 | A1A2 | N/A | A1A1 | A1A2 | A2A2 | A1A1 | A1A2 |
| S15 | A1A2 | A1A2 | A2A2 | A2A2 | A1A2 | A1A1 | A1A1 | A1A2 | N/A | A2A2 | A1A2 | A2A2 | A2A2 | A1A2 |
| S16 | A1A2 | A2A2 | A2A2 | A1A1 | A1A2 | A2A2 | A2A2 | A2A2 | A1A1 | A2A2 | A1A2 | A2A2 | A1A2 | A2A2 |
| S17 | A1A2 | A1A2 | A1A2 | A1A2 | A1A2 | A2A2 | A2A2 | A1A2 | N/A | A1A1 | A1A2 | A2A2 | A2A2 | A1A2 |
| S18 | A1A2 | A1A2 | A1A2 | A1A1 | A2A2 | A2A2 | A1A2 | A2A2 | A1A1 | A1A1 | A2A2 | A2A2 | A1A1 | A1A2 |
| S19 | A1A2 | A1A2 | A2A2 | A1A1 | A1A1 | A2A2 | N/A | A1A2 | N/A | A2A2 | A1A2 | A2A2 | A1A2 | A2A2 |
| S20 | A2A2 | A1A2 | A1A2 | A1A1 | A1A2 | A1A2 | A1A2 | A1A2 | A1A2 | | A1A2 | A2A2 | A1A2 | A1A2 |
| S21 | A1A2 | A2A2 | A1A2 | A1A1 | A1A2 | A2A2 | A1A1 | A1A2 | A1A1 | A1A1 | A1A2 | A2A2 | N/A | A2A2 |

310

320

EP 1 566 760 A2

# FIG. 4

400

| A | B | C | D | E | F | G | H | I | J | K | L | M | N | O | P | Q | R | S | T | U |
|---|---|---|---|---|---|---|---|---|---|---|---|---|---|---|---|---|---|---|---|---|
| S1 | -1 | 0 | 0 | 0 | 1 | 0 | 0 | 1 | 0 | 1 | -1 | 1 | 1 | 1 | 1 | 1 | -1 | 0 | 0 | -1 |
| S2 | -1 | -1 | 0 | 0 | -1 | -1 | 1 | 1 |  | 0 | -1 | 0 | 1 | 1 | 1 | 1 |  | -1 | -1 | -1 |
| S3 |  | -1 | 0 | 0 | 1 | 0 | 1 |  | -1 | 0 | -1 | 0 | 1 | 1 | 1 | 1 | 0 | 1 | 0 | -1 |
| S4 | -1 | 0 | 0 | 0 | -1 | 0 | 1 | 1 | -1 | 1 | -1 | 1 | 1 | 1 | 1 | 0 | 0 | 1 | 0 | -1 |
| S5 | -1 | 1 | 1 | -1 |  | 0 | 0 | 1 | -1 | 1 | 1 | 0 | 1 | 1 | 1 | 0 | 0 | 1 | 0 | -1 |
| S6 | -1 | 0 | 0 | -1 | 1 | 0 | 1 | 1 | 0 | 1 | -1 | 0 | 1 | 1 |  | 0 | 1 | -1 | 0 | -1 |
| S7 | -1 | 0 | 0 | -1 | 0 | -1 | 0 | 1 | 0 | 1 | 1 | 1 | 1 | 0 | 1 | 0 | 1 | 1 | 0 | -1 |
| S8 | -1 | 1 | 0 | 1 | 1 | -1 | 0 | 1 | 1 | 1 | 1 | 0 | 1 | 0 | 1 | 0 | 1 | 0 | 0 | 1 |
| S9 | -1 | 0 |  | 1 | 1 | -1 |  | 1 | 0 | 1 | 1 | 1 | 1 | 0 | 1 | 1 | 1 | -1 | -1 | 0 |
| S10 | -1 | -1 |  | 0 | 1 | -1 | -1 | 1 | -1 | 1 | -1 | 0 | 1 | 0 | 1 | 0 |  | 0 | 0 | -1 |
| S11 | 0 | 0 | 1 | 0 | 0 | -1 | 0 | 1 | -1 | 1 | -1 | 1 | 1 | -1 | 1 | 0 | 1 | 1 | -1 | 0 |
| S12 | -1 | -1 | 0 | 0 | -1 | -1 | 1 | 0 | -1 | 0 | 0 | 1 | 1 | 1 | 0 | 0 | 0 | 0 | 0 | 0 |
| S13 | -1 | 0 | 0 | 1 | 0 | 1 | 0 | 0 | 0 | 0 | 1 | 0 |  | 1 | 0 | 0 | 1 | 0 | 0 |  |
| S14 | -1 | 0 | 0 | 0 | 0 | 0 | 0 | 1 | 1 | 1 | -1 | 0 | 1 | 1 | 0 | 0 | 0 | 0 | -1 |  |
| S15 | -1 | 0 | 0 | 0 | -1 | 0 | 1 | 1 | -1 | 1 | -1 | 0 | 1 | 1 | 0 | 0 | 1 | 0 | 0 | 0 |
| S16 | -1 | 0 | 0 | 0 | 0 | 0 | 0 | -1 | 1 | -1 | -1 | 1 | 1 | 0 | 0 |  | 0 | 1 | 0 | 1 |
| S17 | -1 | -1 | 0 | 0 | 0 | -1 | 1 | 1 | 0 | 1 | -1 | 1 | 1 | -1 | 0 | 0 | 1 | 1 | 0 | -1 |
| S18 | -1 | -1 |  | 0 | 0 | 1 | 1 | 1 |  | 1 | -1 | 1 | 1 | -1 | 0 | 0 | 0 | 0 | -1 | 0 |
| S19 | -1 | 0 | 0 | 0 | 1 | -1 | 1 | 1 | 1 | 1 | -1 | 1 | 1 | -1 | -1 | 0 | 1 | 0 | 0 | -1 |
| S20 |  | 0 | 0 | 0 | 0 | 0 | 0 | 1 | 1 | 1 | 0 | 1 | 1 | 1 | 1 | -1 | 0 | 1 | -1 | 1 |
| S21 | -1 | -1 | 0 | 1 | 1 | 0 | 1 | 1 |  | 1 | -1 | 1 | 1 | 1 | 0 | -1 | -1 | 1 | -1 | 0 |

A2A2 ⟶ -1
A1A2 ⟶ 0
A1A1 ⟶ 1

FIG. 5

| | A | B | C | D | E | F | G | H | I | J | K | L | M | N | O | P | Q | R | S | T | U | V | W | X | Y | Z | AA |
|---|---|---|---|---|---|---|---|---|---|---|---|---|---|---|---|---|---|---|---|---|---|---|---|---|---|---|---|
| 1 | | group | G1 | G2 | G3 | G4 | G5 | G6 | G7 | G8 | G9 | G10 | G11 | G12 | G13 | G14 | G15 | G16 | G17 | G18 | G19 | G20 | G21 | G22 | G23 | G24 | manhattan dis |
| 2 | S1 | norm | 0 | 0 | 1 | 0 | 0 | -1 | 0 | 1 | -1 | 1 | -1 | -1 | 1 | 1 | -1 | 1 | 0 | 1 | 1 | -1 | 0 | 0 | -1 | 0 | 0,708333333 |
| 3 | S2 | norm | -1 | 0 | 0 | 0 | 1 | -1 | 1 | 1 | 1 | 0 | 1 | -1 | 1 | 1 | -1 | -1 | 0 | 1 | 0 | 0 | -1 | -1 | 1 | 0 | 0,625 |
| 4 | S3 | norm | -1 | 1 | 0 | 1 | 1 | -1 | 0 | 1 | 1 | 1 | -1 | 1 | 0 | 1 | 0 | 1 | 0 | 1 | 0 | 0 | 1 | 0 | 1 | 0 | 0,625 |
| 5 | S4 | norm | -1 | -1 | 0 | 0 | 0 | -1 | 1 | 1 | 0 | 1 | -1 | -1 | 1 | 1 | -1 | 0 | 0 | 1 | 1 | 0 | -1 | 1 | 1 | 0 | 0,416666667 |
| 6 | S5 | norm | -1 | -1 | 0 | 0 | -1 | 1 | 1 | 1 | 1 | 1 | -1 | -1 | 1 | 1 | 1 | 0 | 1 | 1 | -1 | 0 | 0 | 1 | 0 | | 0,458333333 |
| 7 | S6 | norm | -1 | 0 | 0 | -1 | -1 | 0 | 0 | 1 | 0 | 0 | -1 | -1 | 1 | 1 | -1 | 1 | 0 | 0 | 1 | -1 | 1 | 1 | 1 | 0 | 0,5 |
| 8 | S7 | norm | -1 | 0 | 0 | 1 | 0 | 0 | 0 | 1 | 0 | 1 | 1 | 1 | 1 | 1 | 0 | 1 | 1 | 0 | 1 | -1 | 0 | 0 | 1 | 0 | 0,416666667 |
| 9 | S8 | norm | -1 | 0 | 0 | 0 | 0 | 0 | 0 | 0 | -1 | 1 | 0 | 1 | ■ | 1 | 1 | 1 | 1 | 1 | 0 | -1 | 1 | 1 | 1 | 0 | 0 |
| 10 | S9 | norm | -1 | 1 | 0 | 0 | 0 | 0 | 1 | 1 | 1 | 0 | -1 | -1 | 1 | 1 | 1 | 0 | -1 | 1 | 0 | -1 | 0 | 1 | 1 | 0 | 0,583333333 |
| 11 | S10 | norm | -1 | 0 | 0 | 0 | 0 | -1 | 0 | 1 | 0 | 0 | -1 | -1 | 1 | 1 | 0 | -1 | 0 | 1 | 0 | 1 | -1 | 0 | -1 | 1 | 0,458333333 |
| 12 | S11 | norm | -1 | 1 | 0 | 1 | 0 | 1 | 1 | 0 | -1 | 0 | -1 | -1 | 1 | 1 | 0 | 0 | 0 | 0 | 0 | 1 | -1 | 0 | 0 | 0 | 0,541666667 |
| 13 | S12 | norm | -1 | 0 | 0 | 0 | 0 | -1 | 1 | 1 | 1 | 0 | -1 | -1 | 1 | 1 | 0 | 1 | -1 | 0 | -1 | 0 | -1 | 1 | 0 | -1 | 0,791666667 |
| 14 | S13 | norm | -1 | 0 | 0 | 1 | 0 | -1 | -1 | 1 | -1 | 0 | 1 | 1 | 1 | 1 | 1 | 0 | 1 | 1 | -1 | -1 | 0 | 0 | 0 | 0 | 0,791666667 |
| 15 | S14 | norm | -1 | 0 | 0 | -1 | 1 | 0 | 0 | 1 | 0 | 1 | -1 | -1 | 0 | 0 | 1 | 0 | 1 | 0 | 0 | 0 | 0 | -1 | 1 | 0 | 0,666666667 |

500

510

# FIG. 6

EP 1 566 760 A2

| | A | B | C | D | E | F | G | H | I | J | K | L | M | N | O | P | Q | R | S | T | U | V | W | X | Y | Z | AA |
|---|---|---|---|---|---|---|---|---|---|---|---|---|---|---|---|---|---|---|---|---|---|---|---|---|---|---|---|
| 1 | | group | G1 | G2 | G3 | G4 | G5 | G6 | G7 | G8 | G9 | G10 | G11 | G12 | G13 | G14 | G15 | G16 | G17 | G18 | G19 | G20 | G21 | G22 | G23 | G24 | manhattan dis |
| 2 | S8 | norm | -1 | 0 | 0 | 0 | 0 | 0 | 0 | 0 | -1 | 1 | 0 | 1 | ■ | 1 | 1 | 1 | 1 | 1 | 0 | -1 | 1 | 1 | 1 | 0 | | 0 |
| 3 | S114 | pat | -1 | 1 | 0 | 0 | 1 | 0 | 1 | 1 | 1 | 0 | -1 | -1 | 1 | 1 | 1 | 1 | 0 | 0 | 0 | 1 | -1 | 1 | -1 | | 0.416666667 |
| 4 | S39 | norm | -1 | 0 | 0 | 0 | 0 | 1 | 0 | 1 | 0 | 0 | 0 | -1 | 1 | 1 | 1 | 1 | 0 | 1 | 0 | -1 | 1 | 1 | 1 | -1 | | 0.541666667 |
| 5 | S84 | pat | -1 | 0 | 0 | 0 | 1 | -1 | 1 | 0 | 0 | 0 | -1 | 0 | 1 | 0 | 1 | 1 | 0 | 0 | 1 | 0 | 0 | 1 | 0 | -1 | | 0.541666667 |
| 6 | S4 | norm | -1 | -1 | 0 | 0 | 0 | -1 | 1 | 1 | 0 | 1 | -1 | -1 | 1 | 1 | -1 | 0 | 0 | 1 | 1 | 0 | -1 | 1 | 1 | 0 | | 0.625 |
| 7 | S7 | norm | -1 | 0 | 0 | 1 | 0 | 0 | 0 | 1 | 0 | 1 | 1 | 1 | 1 | 1 | 0 | 1 | 1 | 0 | 1 | -1 | 0 | 0 | 1 | 0 | | 0.625 |
| 8 | S20 | norm | -1 | 0 | 0 | 0 | 0 | 0 | 0 | -1 | 0 | 0 | -1 | -1 | 1 | 1 | 0 | 0 | 0 | 0 | -1 | 0 | 0 | 1 | 1 | -1 | | 0.5 |
| 9 | S124 | pat | -1 | 0 | 0 | 0 | 0 | -1 | 0 | 1 | 0 | 0 | -1 | 0 | 1 | 1 | 1 | 0 | 1 | -1 | 1 | 1 | 0 | 0 | 0 | | 0.666666667 |
| 10 | S5 | norm | -1 | -1 | 0 | 0 | -1 | 1 | 1 | 1 | 1 | 1 | -1 | -1 | 1 | 1 | 1 | 1 | 0 | 1 | 1 | -1 | 0 | 0 | 1 | | 0.708333333 |
| 11 | S10 | norm | -1 | 0 | 0 | 0 | 0 | -1 | 0 | 1 | 0 | 0 | -1 | -1 | 1 | 1 | 0 | -1 | 0 | 1 | 0 | 1 | -1 | 0 | -1 | 1 | | 0.666666667 |
| 12 | S19 | norm | -1 | 1 | 0 | 0 | 0 | -1 | 1 | 1 | 1 | 1 | -1 | -1 | 1 | 1 | 1 | 1 | 1 | 0 | -1 | 0 | 0 | 1 | 0 | -1 | | 0.541666667 |
| 13 | S45 | norm | -1 | 0 | 0 | 0 | 0 | 0 | 1 | -1 | 0 | 0 | -1 | -1 | 0 | 1 | 1 | 1 | 0 | 0 | 1 | -1 | 0 | 0 | 1 | -1 | | 0.791666667 |
| 14 | S57 | pat | -1 | -1 | 0 | -1 | 0 | -1 | 1 | 1 | 1 | 1 | 1 | 0 | 1 | 1 | 1 | 0 | -1 | 1 | -1 | 0 | 1 | 0 | 1 | 0 | | 0.666666667 |
| 15 | S68 | pat | -1 | -1 | 0 | -1 | 1 | -1 | 0 | 0 | 0 | 0 | -1 | 0 | 1 | 0 | 0 | 0 | 1 | 0 | 0 | 1 | 0 | 1 | | 0.541666667 |
| 16 | S70 | pat | -1 | 0 | 1 | 0 | 1 | 0 | 1 | 1 | 0 | 0 | -1 | 0 | 0 | 1 | 0 | 1 | 1 | 0 | 1 | 0 | 0 | -1 | 0 | | 0.625 |
| 17 | S93 | pat | -1 | 1 | 1 | 0 | 1 | 1 | 0 | 0 | 1 | 0 | -1 | -1 | 1 | 1 | 1 | 0 | -1 | 1 | 0 | 0 | 1 | 1 | -1 | | 0.833333333 |
| 18 | S107 | pat | -1 | 0 | 0 | 0 | 1 | 0 | -1 | 1 | -1 | 1 | -1 | 0 | 1 | 1 | 1 | 0 | 0 | -1 | -1 | -1 | -1 | -1 | 0 | 0 | | 0.666666667 |
| 19 | S128 | pat | -1 | -1 | 0 | 1 | 0 | 0 | -1 | 0 | 1 | 0 | -1 | 0 | 0 | 1 | 0 | 1 | 0 | 0 | 1 | 0 | -1 | 0 | 1 | | 0.458333333 |
| 20 | S129 | pat | -1 | -1 | 0 | 1 | 0 | -1 | 0 | 0 | 0 | 0 | -1 | 0 | 1 | 1 | 0 | 1 | 1 | 0 | -1 | 0 | -1 | 0 | 1 | 0 | | 0.625 |
| 21 | S6 | norm | -1 | 0 | 0 | -1 | -1 | 0 | 0 | 1 | 0 | 0 | -1 | -1 | 1 | 1 | -1 | 1 | 0 | 0 | 1 | -1 | 1 | 1 | 1 | 0 | | 0.583333333 |
| 22 | S18 | norm | -1 | 0 | 0 | 1 | 0 | -1 | 0 | 1 | -1 | 0 | -1 | -1 | 1 | 1 | 0 | -1 | -1 | 0 | 0 | 0 | 0 | 1 | 0 | -1 | | 0.5 |

600 610 620 630 1

FIG. 7

| SAMPLE GROUP CONSTRUCTING UNIT | SIMILARITY COMPARING UNIT | DATA REPLACING UNIT |
|---|---|---|

700      710      720